# EUROPEAN PATENT SPECIFICATION

(11) **EP 2 821 033 B1**
(45) Date of publication and mention of the grant of the patent: **17.08.2016**
(21) Application number: 13755792.2
(22) Date of filing: 15.02.2013
(51) Int. Cl.: A61F 2/82, A61F 2/86, A61F 2/07, A61F 2/848, A61F 2/90

(54) **PROJECTION-TYPE PARTIALLY DUAL-STRUCTURED STENT**
TEILWEISE DOPPELTSTRUKTURIERTER STENT MIT PROJEKTIONSFUNKTION
ENDOPROTHÈSE PARTIELLEMENT À DOUBLE STRUCTURE DE TYPE SAILLIE

(30) Priority: 27.02.2012 KR 20120019526
(43) Date of publication of application: 07.01.2015
(73) Proprietor: National Cancer Center, Goyang-si, Gyeonggi-do 410-769 (KR)
(72) Inventor: KIM, Chan Gyoo, Goyang-si Gyeonggi-do 410-717 (KR)
(74) Representative: Bockhorni & Kollegen
(86) International application number: PCT/KR2013/001181
(87) International publication number: WO 2013/129791

(56) References cited:
- EP-A1- 2 085 050
- WO-A1-99/18887
- WO-A1-2005/058201
- WO-A1-2009/132187
- WO-A1-2011/118081
- WO-A2-03/099167
- KR-A- 20090 083 114
- KR-A- 20110 003 886
- KR-A- 20110 007 825
- KR-A- 20110 007 825
- KR-A- 20110 062 355

## Description

### [Technical Field]

The present disclosure relates generally to a partly projected double-walled stent.

### [Background Art]

In general, stents are lumen expanders that are used to expand a passage/conduit narrowed due to stenosis and are frequently used to treat cancerous or vascular diseases.

Such stents are usually inserted into a stenosed conduit in the human body such as a lung, a blood vessel, a bile duct, a colon, or the esophagus so as to expand the stenosed conduit. However, there is a possibility that these stents may not be maintained in a secured state due to an external force or a pressure of substances flowing through the conduit in the human body, and thus leave an original position. KR 2011 007825 A discloses a double-walled stent having a body configured to have multiple wires woven to be crossed. At least one hook is coupled to the outer circumferential surface of the body. WO 03/099167 A2 also discloses such a stent.

### [Disclosure]

### [Technical Problem]

The present disclosure has been made to provide a technique for allowing a stent to be easily and reliably secured in a conduit in a human body and to present resistance to external forces or pressures applied from the left and right directions.

### [Technical Solution]

According to an aspect of the present disclosure, there is provided a double-walled stent, which includes: a body configured to have multiple wires woven to be crossed, to have an outer circumferential surface formed in a mesh pattern, and to have an interior formed in a hollow cylindrical shape; and at least one protrusion coupled to the outer circumferential surface of the body, and protruding along the outer circumferential surface of the body.

Here, the protrusion includes a first ridge coupled with some of the wires constituting the body and projected from the outer circumferential surface of the body so as to be inclined in one direction, and a second ridge coupled with others of the wires constituting the body and projected from the outer circumferential surface of the body so as to be inclined in the opposite direction, and the first and second ridges may be formed to lie such that they cross over each other.

Further, the double-walled stent may further include a cover configured to enclose an inner circumferential surface of the body.

### [Advantageous Effects]

According to the present disclosure as described above, the first and second ridges of the projection are crossed and projected to be inclined in opposite directions, and thus the partly projected double-walled stent is rigidly secured within a conduit in a human body without being displaced in the left and right directions by an external force.

Also, the number of protrusions can be adjusted according to circumstances so as to simplify a manufacturing process and reduce manufacturing costs.

Further, the cover coupled to the body prevents a lesion portion from penetrating into the partly projected double-walled stent, thus preventing re-stenosis caused by the lesion portion.

### [Description of Drawings]

FIG. 1 is a perspective view of a partly projected double-walled stent according to the present disclosure.
FIG. 2 is an exploded perspective view of the partly projected double-walled stent according to the present disclosure.

### [Best Mode]

Hereinafter, the present disclosure will be described in detail with reference to the accompanying drawings. For clear description, description of known technical portions will be omitted or compressed.

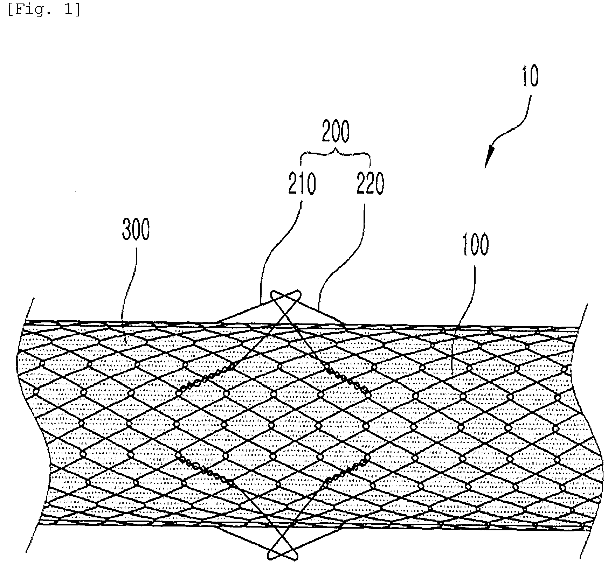

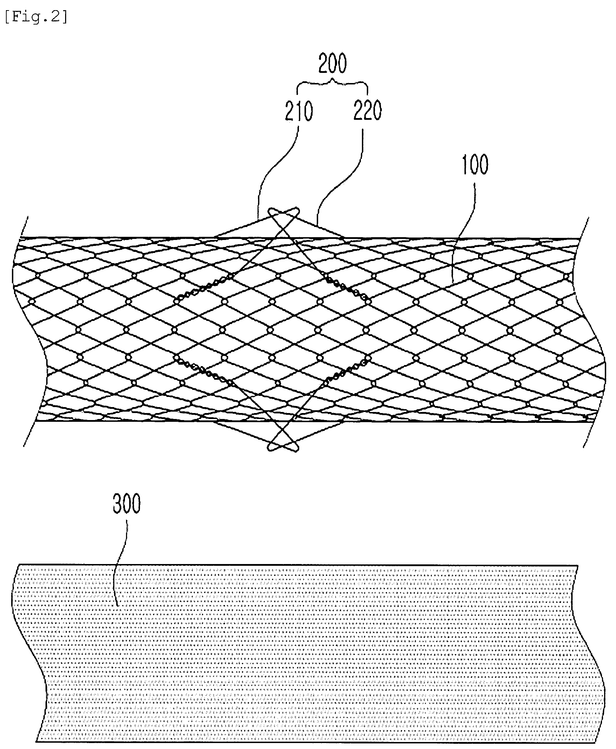

A partly projected double-walled stent 10 according to the present disclosure includes a body 100, at least one protrusion 200, and a cover 300, and will be described with reference to FIGS. 1 and 2.

The body 100 has a hollow cylindrical shape formed by weaving multiple metal wires. Here, the metal wires are crossed and woven in a zigzag pattern, thereby forming a mesh structure on the whole.

The body 100 is inserted into a stenosed inner wall of a conduit in a human body, and expands and secures the stenosed inner wall. As such, the metal wires may be formed of a shape memory alloy such as a nickel-titanium alloy.

The protrusion 200 is a metal wire that is coupled to the metal wires forming an outer circumferential surface of the body and is projected from the outer circumferential surface of the body in a lengthwise direction. In other words, the protrusion 200 generates a frictional force against the conduit inner wall such that the body 100 inserted into the conduit in the human body does not leave the conduit inner wall.

Further, the protrusion 200 is generally made up of a first ridge 210 and a second ridge 220.

The first ridge 210 is a wire that is coupled with some wires constituting the body 100 and is projected from the outer circumferential surface of the body so as to be inclined in one direction. That is, the first ridge 210 is coupled with several wires constituting one side of the body 100, and is projected from the outer circumferential surface of the body in a specific direction, thereby allowing the body 100 to have resistance to an external force that may be applied in the conduit.

For example, when the first ridge 210 is projected from the outer circumferential surface of the body so as to be inclined in the rightward direction, the first ridge 210 generates friction against the inner wall of the conduit. Thereby, the first ridge 210 exerts resistance to an external force or a pressure applied from the right side of the body 100, so that the body 100 can be secured.

The second ridge 220 is a wire that is coupled with some other wires constituting the body 100 and is projected from the outer circumferential surface of the body so as to be inclined in the other direction. That is, the second ridge 220 is coupled with some other wires constituting one side of the body 100, and is projected from the outer circumferential surface of the body in a direction opposite to the first ridge 210, thereby allowing the body 100 to have resistance to an external force that may be applied in the conduit.

For example, when the second ridge 220 is projected from the outer circumferential surface of the body so as to be inclined in the leftward direction, the second ridge 220 generates friction against the inner wall of the conduit. Thereby, the second ridge 220 exerts resistance to an external force or a pressure applied from the left side of the body 100, so that the body 100 can be secured.

If only the first ridge 210 constitutes the protrusion 200, the first ridge 210 fails to exert enough resistance to the external force or the pressure applied from the left side of the body 100, so that the body 100 may not be reliably secured in the conduit.

Therefore, the second ridge 220, which is coupled with several other wires constituting the body 100 and is projected from the outer circumferential surface of the body so as to be inclined in the leftward direction, may be coupled to the body 100.

Further, to maximize the friction between the body 100 and the conduit inner wall to allow the body 100 to be secured in the conduit, the first and second ridges 210 and 220 are formed to intersect each other in an overlapping manner. Here, the first and second ridges 210 and 220 are wires independent of the wires constituting the body 100, and are coupled with the wires constituting the body 100. That is, the first and second ridges 210 and 220 are structurally independent of the body 100.

Thus, the partly projected double-walled stent 10 according to the present disclosure can be stably secured in the human body because the body 100 is not deformed when the external force is applied to the first and second ridges 210 and 220.

In the embodiment of the present disclosure, the protrusions 200 are formed on the respective upper and lower sides of the outer circumferential surface of the body, but the number or positions of the protrusions 200 may be adjusted according to user's need.

Further, in the embodiment of the present disclosure, the first and second ridges 210 and 220 are each formed to extend in a zigzag direction, but the ridges constituting the protrusion 200 may be formed to extend in one direction according to circumstances.

The cover 300 is provided in a hollow cylindrical shape so as to form an internal passage. The cover 300 may be formed of medical polyurethane, silicon-urethane copolymer, silicon, polyamide, polyester, or fluorine resin.

In the embodiment of the present disclosure, the cover 300 is surrounded with an inner circumferential surface of the body, and distinguishes a space between the body 100 and the protrusion 200. In other words, the cover 300 is adapted to cover the metal wires on the inner circumferential surface of the body in a convex state, and to cover the metal wires on the outer circumferential surface of the body in a concave state.

Although the present disclosure has been described through a certain embodiment, it shall be appreciated that various permutations and modifications of the described embodiment are possible by those skilled in the art to which the present disclosure pertains without departing from the scope of the disclosure. Therefore, the scope of the present disclosure shall not be defined by the described embodiment but shall be defined by the appended claims and their equivalents.

## Claims

1. A double-walled stent (10) comprising:
a body (100) configured to have multiple wires woven to be crossed, to have an outer circumferential surface formed in a mesh pattern, and to have an interior formed in a hollow cylindrical shape; and
at least one protrusion (200) coupled to the outer circumferential surface of the body, and projected along the outer circumferential surface of the body
**characterized in that**
the protrusion includes a first ridge (210) coupled with some of the wires constituting the body and projected from the outer circumferential surface of the body so as to be inclined in one direction, and a second ridge (220) coupled with others of the wires constituting the body and projected from the outer circumferential surface of the body so as to be inclined in the opposite direction; and
the first and second ridges are formed to intersect each other in an overlapping manner.

2. The double-walled stent according to claim 1, further comprising a cover (300) configured to enclose an inner circumferential surface of the body.

## Patentansprüche

1. Doppelwandiger Stent (10) umfassend:
einen Körper (100),
der aus einer Mehrzahl an Drähten, die kreuzweise gewoben sind, konfiguriert ist,
der eine äußere Umfangsfläche aufweist, die ein Netzmuster bildet und der ein Inneres aufweist, das als hohle zylindrische Form ausgebildet ist;
und zumindest einen Vorsprung (200), der zu der äußeren Umfangsfläche des Körpers gekoppelt ist und entlang der äußeren Umfangsfläche des Körpers hervorsteht,
**dadurch gekennzeichnet, dass**
der Vorsprung eine erste Erhöhung (210) aufweist, die mit einigen der Drähte gekoppelt ist, welche den Körper bilden und die von der äußeren Umfangsfläche des Körpers hervorsteht, so dass diese in eine Richtung geneigt ist und eine zweite Erhöhung (220), die mit anderen der Drähte gekoppelt ist, welche den Körper bilden und die von der äußeren Umfangsfläche des Körpers hervorsteht, so dass diese in die entgegengesetzte Richtung geneigt ist; und die erste und zweite Erhöhung so ausgebildet sind, dass diese sich gegenseitig in einer überlappenden Weise überkreuzen.

2. Doppelwandiger Stent (10) nach Anspruch 1, **dadurch gekennzeichnet, dass** dieser eine Abdeckung (300) umfasst, die so konfiguriert ist, dass diese eine innere Umfangsfläche des Körpers umfasst.

## Revendications

1. Stent à double paroi (10), comprenant :
un corps (100) configuré pour présenter de multiples fils entrecroisés, pour présenter une surface circonférentielle extérieure formée selon un motif en treillis, et présenter un espace intérieur en forme de cylindre creux ; et
au moins une saillie (200) accouplée à la surface circonférentielle extérieure du corps et faisant saillie le long de la surface circonférentielle extérieure du corps, **caractérisé en ce que**
la saillie comporte une première nervure (210) accouplée à certains des fils constituant le corps et faisant saillie depuis la surface circonférentielle extérieure du corps de manière oblique dans un sens, et une deuxième nervure (220) accouplée à d'autres fils constituant le corps et s'étendant depuis la surface circonférentielle extérieure du corps obliquement dans le sens opposé ; et
les première et deuxième nervures étant formées de manière à se croiser à recouvrement.

2. Stent à double paroi selon la revendication 1, comprenant en outre un capot (300) configuré pour enfermer une surface circonférentielle intérieure du corps.
